# EUROPEAN PATENT SPECIFICATION

(11) **EP 4 223 220 B1**
(45) Date of publication and mention of the grant of the patent: **15.10.2025**
(21) Application number: 22155234.2
(22) Date of filing: 04.02.2022
(51) Int. Cl.: A61B 5/145

(54) **ELECTRONIC CIRCUIT AND ANALYTE SENSOR SYSTEM SUCH AS A GLUCOSE SENSOR SYSTEM AND METHOD OF OPERATING AN ANALYTE SENSOR AND SYSTEM**
ELEKTRONISCHE SCHALTUNG UND ANALYTSENSORSYSTEM WIE Z.B. EIN GLUCOSESENSORSYSTEM UND VERFAHREN ZUM BETRIEB EINES ANALYTSENSORS UND -SYSTEMS
CIRCUIT ÉLECTRONIQUE ET SYSTÈME DE CAPTEUR D'ANALYTE TEL QU'UN SYSTÈME DE CAPTEUR DE GLUCOSE ET PROCÉDÉ DE FONCTIONNEMENT D'UN CAPTEUR ET D'UN SYSTÈME D'ANALYTE

(43) Date of publication of application: 09.08.2023
(73) Proprietor: Roche Diabetes Care GmbH, 68305 Mannheim (DE); F. Hoffmann-La Roche AG, 4070 Basel (CH)
(72) Inventor: Plum, Markus, Mannheim (DE); Fürnrohr, Markus, Stuttgart (DE)
(74) Representative: Grünecker Patent- und Rechtsanwälte PartG mbB

(56) References cited:
- EP-A2- 0 504 835
- GB-A- 1 230 696
- KR-A- 20090 014 449
- US-A1- 2003 097 050
- US-A1- 2009 004 982
- US-A1- 2012 238 890
- US-A1- 2018 159 322
- US-B2- 10 135 242

## Description

The present invention refers to an electronic circuit that is configured to operate an analyte sensor such as a glucose sensor.

US 8,804,291 B2 discloses a system for isolating faults in emergency systems. The document does not disclose miniaturized sensors, in particular glucose sensors.

US 2008/0249385 A1 discloses a continuous intravenous analyte monitoring system including different isolated circuits. Also US 2003/097050 A1 discloses relevant prior art.

Such glucose sensors are known from the prior art where these glucose sensors have electrodes which can be inserted or implanted into a patient. Such sensors may include two or three or more electrodes that are specifically designed in order to change electric properties such as a resistance depending on an analyte concentration such as depending on a glucose concentration. For that purpose electrodes with a membrane are known.

These sensors are suitable for measuring the glucose concentration in an interstitial body fluid. These sensors can also be configured to measure the glucose concentration in blood. An electronic circuit is also known from the prior art configured to operate such an analyte sensor in order to obtain a measurement of an electrical potential or a change of an electric property of the sensor indicative of the glucose concentration of a patient.

As the electrodes are inserted or implanted into the patient and the electronic circuit operates with a certain voltage, it is contemplated to have some safety feature that prevents uncontrolled currents from flowing through the electrodes and through the patient.

The object of the present invention is therefore to provide an electronic circuit configured to operate an analyte sensor that provides certain safety with respect to controlling current flow through the patient.

Further, it is an object of the present invention to provide a corresponding analyte sensor system which comprises an electronic circuit and analyte sensor and it is an object of the invention to provide a method for operating such an analyte sensor or of operating an analyte sensor system.

The object of the present invention is solved by the electronic circuit of claim 1, the analyte sensor system of claim 9 and the method of claim 10.

The electrodes to which the first and second electrical connection of the electronic circuit may be connected may be electrodes suitable for being inserted or implanted into a patient. They may be inserted or implanted individually or together. The electrodes may be suitable for measuring or detecting an analyte concentration (such as glucose) in an interstitial fluid of the patient or in the patient's blood.

One or two of the electrodes may be provided with an enzyme that selectively reacts with an analyte such as glucose. The electrodes may be configured such that depending on the analyte concentration they change their electrical properties such as a resistance or impedance measured between two of the electrodes.

Accordingly, the electronic circuit has a first and second electrical connection. The electrodes of an analyte sensor can be connected to these first and second electrical connections. The electronic circuit may nevertheless also have more than a first and a second electrical connection such as three or four or more electrical connections. It may be possible that e.g. two or three electrical connections are provided in order to contact one and the same electrode of an analyte sensor in order to provide some redundancy. Also, it is possible to have an analyte sensor with more than two electrodes and then more than two electrical connections are necessary anyhow.

The electronic circuit has a voltage source such as a battery and a common potential conductor section which is electrically connected to one pole of the voltage source such as to the minus pole or 0V pole of the voltage source. This common potential conductor section comprises or is made of an electrically conductive material such as a metal or metal alloy. It may comprise or be made of copper or a metal alloy comprising copper and/or tin. It provides the same electric potential to different portions of the electronic circuit and may be considered as a ground of the electronic circuit. The common potential conductor section may for example provide the common potential of one pole of the battery and a (nominal ground) connection of an integrated circuit (which may include an operational amplifier, OpAmp) within the electronic circuit. The common potential conductor section may be electrically provided on a ground potential or it may be on a floating potential but still providing a common potential to different portions of the sensor (like a ground) such as the potential of the pole of the voltage source. The common potential conductor section may e.g. be given by or include a conductor path, a conductor strip or a conductor patch of a circuit board.

The electric potential of the common potential conductor section is called common potential in the following.

With the voltage source, it is possible to provide an electrical potential different to the common potential to the first electrical connection. For this purpose, electronic circuitry may be provided in the electronic circuit that regulates and controls the electric potential provided to the first electrical connection in comparison to the common potential. The electronic circuitry defines or regulates or controls the potential difference between the common potential and the potential provided to the first electrical connection.

The second electrical connection is connected to the common potential conductor section through one or more connection paths (in the following these connection path are called common potential connection paths). However, none of those common potential connection paths connects the second electrical connection to the common potential through fewer than three or more series-connected electronic components.

Thereby it is achieved that, if in one of the common potential connection paths, one or two of the series-connected electronic components fail e.g. by being short-circuited or having a short circuit, then the third electronic component is still able to limit the current that flows from the second electrical connection to the common potential conductor section and thereby to limit the current that flows from the first electrode to the second electrode, i.e. through the patient.

The current that flows through the second electrical connection and is limited by the configuration of the common potential connection paths may be a DC current or an AC current or a current having both a DC component and an AC component.

At least in one, two, three or more than three or in all common potential connection paths, the electronic components in each of those common potential connection paths are of the same type. The types are either e.g. being an inductive component, a capacitive component, a resistive component or a diode component or another electronic component. An inductive component is provided in order to provide an electronic inductivity. The capacitive component is provided in order to provide an electronic capacity. The resistive component is provided in order to provide an ohmic resistance. The diode component provides the function of a diode, i.e. blocking current flow in one direction but allowing a current flow in the other direction of the two connections of a diode.

Other types of components may be considered as well.

In case that, for example in one of the common potential connection paths, all electronic components are resistive components, the three components together that are series-connected provide a required resistance for the circuit to function properly. If one of the resistive components is short-circuited, e.g. by a failure of the resistive component, the measurement of the potential of the electrode or of the electric property of the sensor and thereby of the analyte concentration may or may not be reasonable, reliable or correct any more but nevertheless an uncontrolled flow of current through the patient can be avoided. This holds even if two of the three resistive components fail in the sense that both are short-circuited or deviate with their ohmic resistance from the nominal ohmic resistance they are supposed to provide, in particular deviating from their nominal ohmic resistance to a lower ohmic resistance. The circuit may be designed such that even in case of a failure of one or two components in the common potential connection paths the measurement of the analyte concentration may still be reasonable. This may or may not be done e.g. by a calibration of the sensor with another separate analyte concentration measurement method.

Similar considerations apply to a capacitive component, an inductive component or a diode component. For example, three diodes may be provided in a series and if one of the diodes fails in the sense that it allows a current to flow through it in the direction the diode is supposed to block the current, then the other two diodes can still fulfil this function. This holds even if two of the diodes fail and only the third diode blocks the current flow. The analyte sensor works as normal and can measure the analyte concentration independent of the number of failed diodes as long as one diode is blocking the current as a non-failed diode is supposed to block.

For this purpose, also all of the electronic components of the same type in one common potential connection path have the same nominal specification concerning their electric function such as the same nominal resistance value, the same nominal capacity value, the same nominal inductivity value or the same diode specification.

If, e.g. in total a resistance of 150 Ohm is to be provided then the n series-connected components (with n being for example three) can be provided with each a nominal resistance value of 150 times 1/n Ohm (with n being three, the resistance being 50 Ohm).

Optionally, in different common potential connection paths, electronic components of different types are provided, the types being an inductive component, a capacitive component, a resistive component and a diode component.

Thereby it is possible to provide different kinds of electric functions in the different common potential connection paths.

In order to avoid undesired current flows such as e.g. by leakage currents, it is optionally possible to provide that a distance is provided from any series-connected electronic components in one, two, three or more or all common potential connection paths to any other electronic component that is not directly electrically connected to the series-connected electronic component. This distance may be at least 0.25 mm (millimeters), 0.4 mm, 0.5 mm, 0.6 mm or 0.75 mm. The distance between neighboring series-connected electronic components (directly connected series-connected electronic components, without any other component between them apart from the electric connection) may be more or less than that value as they are conductively connected to each other anyway on purpose.

For that reason of avoiding undesired current flows, it is also provided that the distance from all series-connected electronic components of all common potential connection paths to any other electronic component that is not directly connected may be at least 0.25 mm, 0.4 mm, 0.5 mm or 0.6 mm or 0.75 mm.

The design of the electronic circuit may be such that via short-circuit of all but one of the series-connected electronic components in each common potential connection path, the resulting current through the patient is limited to a predefined current threshold. This current threshold may be equal to or less than 25µA, 50µA or 75µA. Such threshold values correspond to the DC current in case the current has only a DC component and no AC component. In case the current has only an AC component, the indicated threshold current refers to the peak current. In case the current has an AC and a DC component, the threshold refers to the DC component plus the peak of the AC component.

Optionally, the design is such that the resulting current (in case of the above mentioned short circuit of all but one of the series-connected electronic components in each common potential connection path) is less than the predefined current threshold (of e.g. less than or equal to 50µA) even if the electric potential provided to the first electrical connection is equal to the maximum potential available in the electronic circuit such as for example the potential provided by a voltage source such as a battery that powers the electronic circuit and/or even if the first and second electrical connections are short circuited e.g. by an electrical contact of the two electrodes.

An analyte sensor system comprises the electronic circuit as described above or below and an analyte sensor such as a glucose sensor. The analyte sensor may be provided together with the electronic circuit as a kit but be separated from each other, which means that the analyte sensor and the electronic circuit are not (yet) connected. Also, the analyte sensor system may refer to the electronic circuit being provided in combination with the analyte sensor which is connected to the electronic circuit.

The invention also refers to a method of operating an analyte sensor with an electronic circuit described as above or below or may refer to a method of operating an analyte sensor system as described above or below.

In order to provide a better understanding of the invention, the following figures are provided. The figures show:
Figure 1: a schematic explanation of the different items of an electronic circuit.
Figure 2: a detail of the different common potential connection paths and the series-connected electronic components.
Figure 3: a three-dimensional view of surface-mounted electronic components on a circuit board.

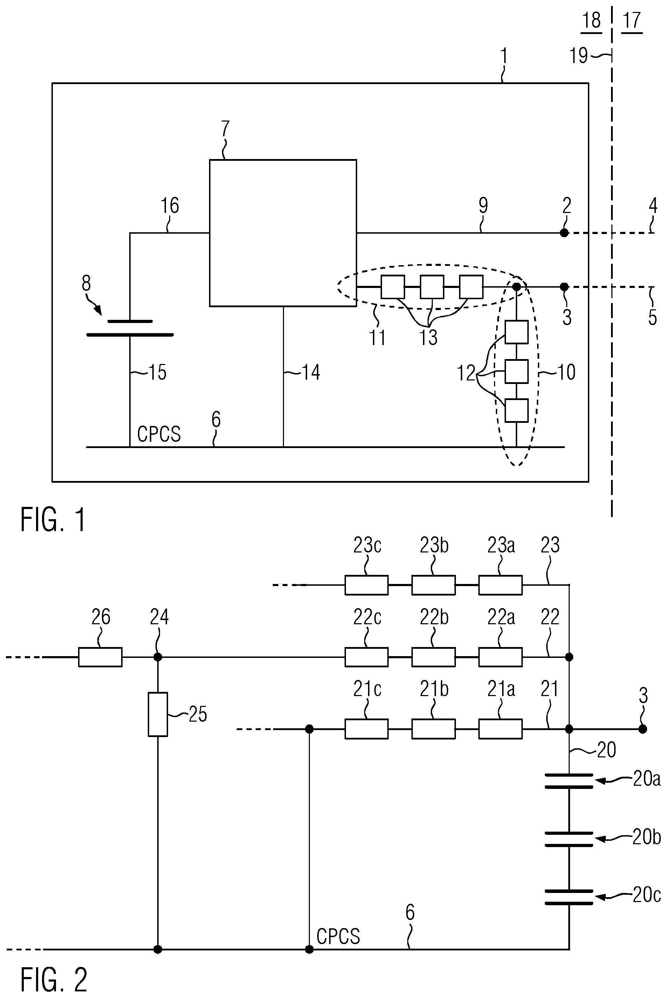

Figure 1 shows schematically items of an electronic circuit 1. The electronic circuit 1 has a first and second electrical connection 2, 3 respectively. To those first and second electrical connections 2, 3, electrodes 4, 5 of an analyte sensor can be connected. One of the two electrodes at least is provided with a specific design which allows it to react specifically to the analyte in question that is to be measured or detected. Such an analyte may be glucose, for example. Commonly, one of the two electrodes 4, 5 is called a working electrode and the other one may be called a counter electrode. The electrodes 4 and 5 are typically provided jointly as one or inside of one analyte sensor, although in Fig. 1 they are drawn as separate items.

For example, electrode 4 could be the working electrode and electrode 5 the counter electrode. Accordingly, electrical connection 2 would be the connection for the working electrode 4 and electrical connection 3 would be the electrical connection for the counter electrode 5.

Electrodes 4 and 5 can be inserted or implanted into the body of a patient. The region inside of the patient is marked with reference numeral 17 and the region outside is marked with reference numeral 18. The skin of the patient is noted with reference numeral 19. The electronic circuit 1 may be provided on the skin 19 of the patient. The first and second electrodes 4, 5 are designed to penetrate the skin 19 and to be provided each with at least one of their ends in the body of the patient.

The analyte sensor with the electrodes 4, 5 can be disconnected from the electronic circuit as the electronic circuit can be used several times while the analyte sensor may be exchanged after a while. It may also be used only once and/or the electronic circuit may be used only once.

The electronic circuit 1 has a voltage source 8 here provided as a battery that provides a voltage V such as e.g. 1.5 V, 2 V or 3 V or 5 V or the like. One of the two battery poles is connected to common potential conductor section 6 (CPCS) with an electrical connection 15. This common potential conductor section may be called ground even if the potential of the common potential conductor section may be on a floating potential or may be connected to ground potential through an external connection to an external ground. The other battery pole is connected to one or more electronic components or one or more electronic integrated circuits. Such an integrated circuit is schematically shown in Figure 1 with reference numeral 7. The integrated circuit 7 may itself be connected to the common potential conductor section 6 by an electrical connection 14. The integrated circuit 7 may further provide an electrical potential to the electrical connection 2 via the connection 9. The electric potential provided to the electrical connection 2 may be as high as the voltage provided by the voltage source 8 or it may be lower. The electric potential provided to the electrical connection 2 can be adjusted by the integrated circuit 7. The integrated circuit 7 may for example comprise an operational amplifier. This operational amplifier can be involved in adjusting the electric potential (in comparison to the common potential conductor section) provided to the electrical connection 2.

The second electrical connection 3 is connected to the common potential conductor section 6 via the common potential connection path 10. The second electrical connection 3 may additionally be connected to the common potential conductor section 6 by another common potential connection path as shown in Figure 1 wherein such a second common potential connection path is given by reference numeral 11 which has several series-connected electrical components 13 which connect the electrical connection 3 with the integrated circuit 7 which itself is connected to the common potential conductor section by connection 14. While in Figure 1, two common potential connection paths 10, 11 are shown, there may be only one common potential connection path or there may be more than two common potential connection paths. In the common potential connection path 10, there are provided three series-connected electronic components 12. In a second and optional common potential connection path 11, there are provided three series-connected electronic components 13.

In case of a failure of e.g. the integrated circuit 7 and the integrated circuit 7 provides an uncontrolled voltage to electrical connection 2, such voltage will be in its worst case as high as the voltage provided by the voltage source 8. The three electronic components 12 in the common potential connection path 10 are chosen such that even if two of these three electronic components 12 are short-circuited by a component failure or other failures, one of those electronic components 12 is capable of maintaining the current through the electrical connection 3 to less than a predefined threshold such as a threshold of less than or equal to 50µA in case the full electrical potential of the voltage source 8 is applied to the first electrical connection 2.

Thereby in case of a component failure the current that goes through the patient is limited to the predefined threshold of e.g. less than or equal to 50µA.

The predefined threshold of less than or equal to 50µA is mentioned here exemplarily only. The predefined threshold can also be less than or be equal to 25µA or 100µA or any other value as desired.

The same considerations apply to the common potential connection path 11 with its three series-connected electronic components 13. Even in case two of the three components fail, only one of the three components 13 is capable of limiting the current through electrical connection 3 to the predefined threshold such as less than or equal to 50µA.

Figure 2 shows in more detail the view of possible common potential connection paths provided for the electrical connection 3. In total, in Figure 2, four common potential connection paths 20, 21, 22 and 23 are shown. Each of those common potential connection paths 20, 21, 22, 23 comprises at least three series-connected electronic components. In common potential connection path 20, these are the components 20a, 20b and 20c, in common potential connection path 21 these are the components 21a, 21b and 21c. The same applies to the other two common potential connection paths 22 and 23.

In Figure 1, two common potential connection paths 10, 11 are shown and in Figure 2, four common potential connection paths 20, 21, 22 and 23 are shown. However, that number of common potential connection paths can also be another number of common potential connection paths such as 1, 3, or more than 4.

In Figs. 1 and 2, it is shown that none of the common potential connection paths connects the second electrical connection to the common potential conductor section 6 through fewer than three or more series-connected electronic components. This means that by a single failure of one or two electronic components, the current through electrical connection 3 is always limited to a predefined threshold (limit) of as less than or equal to 50µA.

In Figure 2, the common potential connection path 20 consists of three capacitors 20a, 20b and 20c. In the common potential connection paths 21, 22 and 23, the respectively three series-connected electronic components are all resistors.

Other components such as diodes or inductivities however can also be provided in one or more of the common potential connection paths.

The three capacitors 20a, 20b and 20c may have identical nominal capacity values. For example, each capacity may be specified to be 500 nF. It may also be 680 or 750 nF or 1 µF. Each of those capacitors itself alone is capable of sufficiently isolating electrical connection 3 against the common potential conductor section 6 in case of a failure of two of the three components 20a, 20b, 20c. In this regard, for example, consider that electrical components 20a and 20b have a failure and are short-circuited. The resulting capacitance provided between the second electrical connection 3 and common potential conductor section 6 is then only given by the capacitance of capacitor 20c. This would be sufficient to limit the current to the predefined threshold. In particular, any of the three capacitors is capable of suppressing a DC current even if two of the other capacitors are short circuited.

In the above example, the capacitance of the three capacitors 20a, 20b and 20c are the same. The capacitance values however may also be different with respect to each other.

Common potential connection path 21 also provides a connection of electric connection 3 to the common potential conductor section 6 via the three resistors 21a, 21b and 21c.

Each one of the resistors 21a, 21b and 21c alone is great enough in order to limit the current through electrical connection 3 and through that resistors to the common potential conductor section 6 to be not more than the pre-defined threshold of e.g. less than or equal to 50µA. The nominal value of the resistance of each of these three resistors 21a, 21b and 21c may be identical such as e.g. 100 kΩ or 75 kΩ or any other value. The nominal resistance values of the three resistors may however also be different with respect to each other.

The same applies to the other two common potential connection paths 22 and 23 shown in Figure 2.

While the common potential connection paths 20 and 21 connect electrical connection 3 through the three series-connected electronic components directly with the common potential conductor section, that may be different for other common potential connection paths such as shown for common potential connection paths 22 and 23. For example, common potential connection path 22 comprises the three series-connected electronic components 22a, 22b and 22c which connect electrical connection 3 to the node 24. The node itself is then connected to the common potential conductor section 6 through a further resistance or other electronic component 25. Node 24 may also be connected through another electronic component 26 to further circuitry of the electronic circuit.

The same applies to common potential connection path 23. This common potential connection path can, for example, be connected to the integrated circuit 7 or it may be connected to an operational amplifier within the integrated circuit 7 or the like.

As explained beforehand with respect to Figure 1, also in Figure 2 none of the common potential connection paths connects the second electrical connection 3 to the common potential conductor section 6 through fewer than three or more series-connected electronic components. In other words, there is no connection from the electrical connection 3 to the common potential conductor section 6 through fewer than three electronic components. Thereby each of such three electronic components is provided with a nominal value concerning their electric function that would not allow a current flow of more than the pre-defined limit of e.g. less than or equal to 50µA through electrical connection 3, even if the full voltage of a voltage supply 8 is provided to electrical connection 2 (see Figure 1).

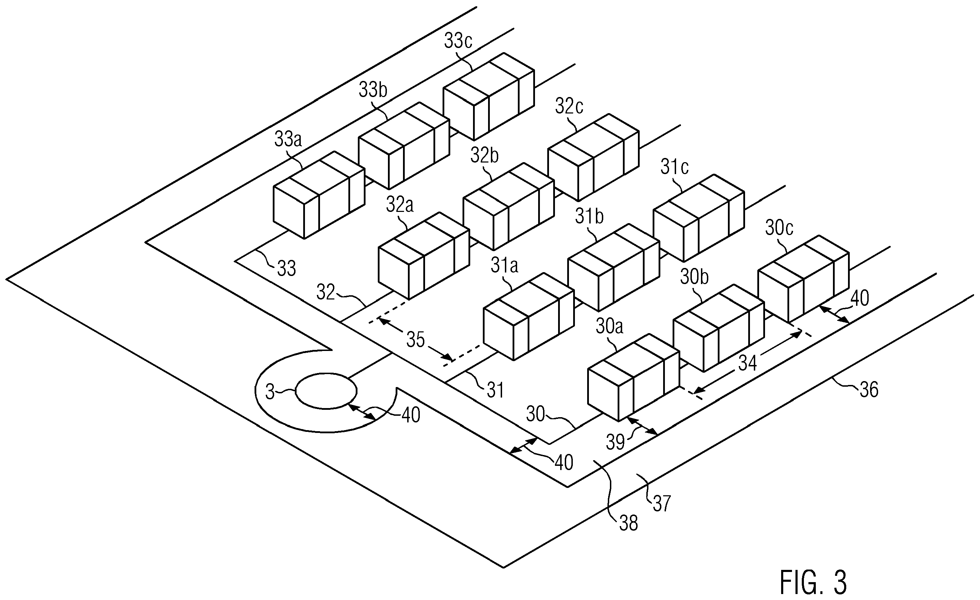

In Figure 3, an example of surface-mounted electronic components is shown. In total, four common potential connection paths 30, 31, 32 and 33 are shown, each of which comprises at least three in-series-connected electronic components. For example, common potential connection path 30 comprises the three series-connected electronic components 30a, 30b and 30c. The three series-connected electronic components are provided at a distance to non-neighboring electronic components. The distance 34 between two of the series-connected electronic components such as between 30a and 30c, which are not neighboring electronic components, may be at least 0.25 mm or at least 0.4 mm or at least 0.5 mm or at least 0.6 mm or at least 0.75 mm. Further, the distance 35 between electronic components of different common potential connection paths may be at least 0.25 mm or at least 0.4 mm or at least 0.5 mm or at least 0.6 mm or at least 0.75 mm such as the distance between electronic component 31b and 32b. The series-connected electronic components 30a and 30b are neighboring series-connected electronic components as they are directly electrically connected to each other. Between the neighboring series-connected electronic components there is no other electronic component between them electrically. This means that they are directly electrically connected with each other and between them is electrically only the electrical connection such as a copper trace and/or solder material. The same applies to series-connected electronic components 30b and 30c and the electronic components 30a and 31a which are directly connected to each other.

While in the figures series-connected electronic components are always shown as being provided on a straight line, they need not be provided in such a way as they also can be provided on a bent or folded or zig-zag or U-shaped line. In case of a U-shape one of the three series-connected electronic components is each provided on a leg of the U. The distance between non-neighboring series connected electronic components is also at least 0.25 mm, 0.4 mm, 0.5 mm, 0.6 mm or 0.75 mm in case the series-connected electronic components are not provided on a straight line but on a line of any other shape as mentioned above.

Optionally, a separation or distance 34, 35 is provided between all the electronic components of each of the common potential connection paths such as each of the common potential connection paths 30, 31, 32 and 33 and any non-neighboring series-connected electronic component.

The electronic components of two distinct common potential connection paths are optionally provided at a distance 35. Furthermore, the distance 35 between the electronic components of two distinct common potential connection paths can be at least 0.25 mm, 0.4 mm, 0.5 mm, 0.6 mm or 0.75 mm.

Providing the distances 34 and 35 prevent leakage currents to be of such substantial amount that the pre-defined limit of e.g. less than or equal to 50µA is surpassed in case of a component failure of two of the three series-connected electronic components of each common potential connection paths.

Furthermore, a distance 39 between each of the series-connected electronic components of each of the common potential connection paths from a common potential conductor section 37 or of a conductive patch 37 being connected to the voltage source 8 is provided. That distance may be at least 0.25 mm, 0.4 mm, 0.5 mm or at least 0.6 mm or at least 0.75 mm.

In Figure 3, such a common potential plane or conductive patch 37 is shown on a circuit board 36 surrounding the common potential connection paths 30, 31, 32 and 33. Between the common potential connection paths 30, 31, 32 and 33 and the electrical connection 3 on the one side and the common potential conductor section 37 or the conductive patch 37 on the other side is a conductor free area 38. This conductor free area 38 has an extension 40 in the direction parallel to the circuit board 36 of at least 0.25mm or at least 0.4 mm or at least 0.5 mm or at least 0.6 mm or at least 0.75 mm in order to prevent or sufficiently suppress undesired leakage currents.

## Claims

1. Electronic circuit (1) configured to operate an analyte sensor (4, 5), such as a glucose sensor, the circuit having at least a first and a second electrical connection (2, 3) configured to be connected to a first and a second electrode (3, 4) of the analyte sensor respectively,
wherein the electronic circuit (1) has a voltage source (8) and a common potential conductor section (6) electrically provided on a potential of a pole of the voltage source,
wherein with the voltage source (8) an electric potential different to the potential of the common potential conductor section (6) can be provided to the first electrical connection (2);wherein the second electrical connection (3) is connected to the common potential conductor sec-tion (6) through one or more common potential connection paths (10, 11, 20, 21, 22, 23, 30, 31, 32, 33) and wherein none of the common potential connection paths (10, 11, 20, 21, 22, 23, 30, 31, 32, 33) connects the second electrical connection (3) to the common potential conductor sec-tion (6) through fewer than three series-connected electronic components (12, 13,...);
**characterised in that**
at least in one, two, three or more or all common potential connection paths (10, 11, 20, 21, 22, 23, 30, 31, 32, 33) the electronic components (12, 13,...) in each common potential connection path (10, 11, 20, 21, 22, 23, 30, 31, 32, 33) are of the same type, the type being selected from a group of types, the group of types including for example an inductive component, a capacitive component, a resistive component and/or a diode component; and
wherein all electronic components (12, 13,...) of the same type in one common potential connection path (10, 11, 20, 21, 22, 23, 30, 31, 32, 33) have the same nominal specification concerning their electric function, such as the same nominal resistance value, the same nominal capacity value, the same nominal inductivity value or the same diode specification.

2. Electronic circuit as of claim 1, wherein in different common potential connection paths (10, 11, 20, 21, 22, 23, 30, 31, 32, 33) electronic components (12, 13) of different types are provided, the types being selected from a group of types, the group of types including for example an inductive component, a capacitive component, a resistive component and/or a diode component.

3. Electronic circuit as of any of claims 1 or 2, wherein the series-connected electronic components (12, 13, ...) of one, two, three, or more or all common potential connection paths (10, 11, 20, 21, 22, 23, 30, 31, 32, 33) are provided as components on a circuit board (36), optionally as sur-face-mounted components (30a,..., 33c).

4. Electronic circuit as of any of claims 1 to 3, wherein all series-connected electronic components (12, 13,...) of all common potential connection paths (10, 11, 20, 21, 22, 23, 30, 31, 32, 33) are provided on one circuit board (36), optionally as surface-mounted components (30a,...,33c).

5. Electronic circuit as of any of claims 1 to 4, wherein the distance (34, 35) from any series-connected electronic component (30a .. 33c) in one, two, three or more or all common potential connection paths to any other electronic component of the electronic circuit which is not a directly connected series connected electronic component is at least 0.25 mm or 0.4 mm or 0.5 mm or 0.6 mm or 0.75 mm.

6. Electronic circuit as of any of claims 1 to 5, wherein the distance (34, 35) from all series-connected electronic components (30a .. 33c) of all common potential connection paths to any other electronic component which is not a directly connected series-connected electronic component is at least 0.25 mm, or 0.4 mm, or 0.5 mm or 0.6 mm or 0.75 mm.

7. Electronic circuit as of any of claims 1 to 6, wherein by a short circuit of all but one of the series-connected electronic components(12, 13,... 33c) in each common potential connection path (10, 11, 20, 21, 22, 23, 30, 31, 32, 33) the resulting current through the first or second electrical connection (2, 3) is limited to less than a predefined threshold, the threshold being equal to or less than 25µA, 50µA or 75 µA.

8. Electronic circuit as of claim 7, wherein the resulting current is limited to less than the predefined threshold in case the electric potential provided to the first electrical connection (2) is equal to the maximum potential available in the electronic circuit such as for example the potential provided by a voltage source or battery (8) that powers the electronic circuit (1) and/or in case the first and second electrical connections are short circuited e.g. by an electrical contact of the two electrodes.

9. Analyte sensor system (1, 4, 5) comprising the electronic circuit (1) as of any of claims 1 to 8 and an analyte sensor (4, 5) such as a glucose sensor, wherein the analyte sensor (4, 5) is provided with the electronic circuit as a kit and the analyte sensor (4, 5) and the electronic circuit (1) are not connected, or the analyte sensor (4, 5) is connected to the electronic circuit (1).

10. Method of operating an analyte sensor (4, 5) with an electronic circuit (1) as of any of claims 1 to 8 or of operating an analyte sensor system (1, 4, 5) as of claim 9.

## Patentansprüche

1. Elektronische Schaltung (1), die dafür ausgebildet ist, einen Analytsensor (4, 5), wie einen Glukosesensor, zu betreiben, wobei die Schaltung mindestens eine erste und eine zweite elektrische Verbindung (2, 3) aufweist, die dafür ausgebildet sind, mit einer ersten bzw. einer zweiten Elektrode (3, 4) des Analytsensors verbunden zu werden,
wobei die elektronische Schaltung (1) eine Spannungsquelle (8) und einen gemeinsamen Potentialleiterabschnitt (6), der elektrisch an einem Potential eines Pols der Spannungsquelle vorgesehen ist, aufweist,
wobei mit der Spannungsquelle (8) der ersten elektrischen Verbindung (2) ein elektrisches Potential, das verschieden ist von dem Potential des gemeinsamen Potentialleiterabschnitts (6), zugeführt werden kann; wobei die zweite elektrische Verbindung (3) über einen oder mehrere gemeinsame(n) Potentialverbindungsweg(e) (10, 11, 20, 21, 22, 23, 30, 31, 32, 33) mit dem gemeinsamen Potentialleiterabschnitt (6) verbunden ist und wobei keiner der gemeinsamen Potentialverbindungswege (10, 11, 20, 21, 22, 23, 30, 31, 32, 33) die zweite elektrische Verbindung (3) über weniger als drei in Reihe geschaltete elektronische Komponenten (12, 13,...) mit dem gemeinsamen Potentialleiterabschnitt (6) verbindet;
**dadurch gekennzeichnet, dass** mindestens in einem, zwei, drei oder mehr oder allen gemeinsamen Potentialverbindungsweg(en) (10, 11, 20, 21, 22, 23, 30, 31, 32, 33) die elektronischen Komponenten (12, 13,...) in jedem gemeinsamen Potentialverbindungsweg (10, 11, 20, 21, 22, 23, 30, 31, 32, 33) vom selben Typ ist/sind, wobei der Typ aus einer Gruppe von Typen ausgewählt ist, wobei die Gruppe von Typen beispielsweise eine induktive Komponente, eine kapazitive Komponente, eine ohmsche Komponente und/oder eine Diodenkomponente einschließt; und
wobei alle elektronischen Komponenten (12, 13,...) vom selben Typ in einem gemeinsamen Potentialverbindungsweg (10, 11, 20, 21, 22, 23, 30, 31, 32, 33) dieselbe Nennspezifikation bezüglich ihrer elektrischen Funktion, wie denselben Nennwiderstandswert, denselben Nennleistungswert, denselben Nenninduktivitätswert oder dieselbe Diodenspezifikation, aufweisen.

2. Elektronische Schaltung nach Anspruch 1, wobei in verschiedenen gemeinsamen Potentialverbindungswegen (10, 11, 20, 21, 22, 23, 30, 31, 32, 33) elektronische Komponenten (12, 13) verschiedener Typen vorgesehen sind, wobei die Typen aus einer Gruppe von Typen ausgewählt sind, wobei die Gruppe von Typen beispielsweise eine induktive Komponente, eine kapazitive Komponente, eine ohmsche Komponente und/oder eine Diodenkomponente einschließt.

3. Elektronische Schaltung nach einem der Ansprüche 1 oder 2, wobei die in Reihe geschalteten elektronischen Komponenten (12, 13,...) von einem, zwei, drei oder mehr oder allen gemeinsamen Potentialverbindungsweg(en) (10, 11, 20, 21, 22, 23, 30, 31, 32, 33) als Komponenten auf einer Leiterplatte (36), gegebenenfalls als oberflächenmontierte Komponenten (30a,..., 33c), vorgesehen sind.

4. Elektronische Schaltung nach einem der Ansprüche 1 bis 3, wobei alle in Reihe geschalteten elektronischen Komponenten (12, 13,...) aller gemeinsamen Potentialverbindungswege (10, 11, 20, 21, 22, 23, 30, 31, 32, 33) auf einer Leiterplatte (36), gegebenenfalls als oberflächenmontierte Komponenten (30a,..., 33c), vorgesehen sind.

5. Elektronische Schaltung nach einem der Ansprüche 1 bis 4, wobei der Abstand (34, 35) von einer in Reihe geschalteten elektronischen Komponente (30a .. 33c) in einem, zwei, drei oder mehr oder allen gemeinsamen Potentialverbindungsweg(en) zu einer anderen elektronischen Komponente der elektronischen Schaltung, die keine direkt geschaltete in Reihe geschaltete elektronische Komponente ist, mindestens 0,25 mm oder 0,4 mm oder 0,5 mm oder 0,6 mm oder 0,75 mm beträgt.

6. Elektronische Schaltung nach einem der Ansprüche 1 bis 5, wobei der Abstand (34, 35) von allen in Reihe geschalteten elektronischen Komponenten (30a .. 33c) aller gemeinsamen Potentialverbindungswege zu einer anderen elektronischen Komponente, die keine direkt geschaltete in Reihe geschaltete elektronische Komponente ist, mindestens 0,25 mm oder 0,4 mm oder 0,5 mm oder 0,6 mm oder 0,75 mm beträgt.

7. Elektronische Schaltung nach einem der Ansprüche 1 bis 6, wobei durch einen Kurzschluss aller, außer einer der in Reihe geschalteten elektronischen Komponenten (12, 13,... 33c) in jedem gemeinsamen Potentialverbindungsweg (10, 11, 20, 21, 22, 23, 30, 31, 32, 33) der resultierende Strom über die erste oder zweite elektrische Verbindung (2, 3) auf weniger als einen vordefinierten Schwellenwert begrenzt wird, wobei der Schwellenwert kleiner oder gleich 25 µA, 50 µA oder 75 µA ist.

8. Elektronische Schaltung nach Anspruch 7, wobei der resultierende Strom auf weniger als den vordefinierten Schwellenwert begrenzt wird, wenn das der ersten elektrischen Verbindung (2) zugeführte elektrische Potential gleich dem maximalen Potential ist, das in der elektronischen Schaltung verfügbar ist, wie beispielsweise das Potential, das von einer Spannungsquelle oder Batterie (8) geliefert wird, die die elektronische Schaltung (1) speist, und/oder wenn die erste und zweite elektrische Verbindung, z. B. durch einen elektrischen Kontakt der beiden Elektroden, kurzgeschlossen werden.

9. Analytsensorsystem (1, 4, 5), umfassend die elektronische Schaltung (1) nach einem der Ansprüche 1 bis 8 und einen Analytsensor (4, 5), wie einen Glukosesensor, wobei der Analytsensor (4, 5) mit der elektronischen Schaltung als ein Kit vorgesehen ist und der Analytsensor (4, 5) und die elektronische Schaltung (1) nicht verbunden sind oder der Analytsensor (4, 5) mit der elektronischen Schaltung (1) verbunden ist.

10. Verfahren zum Betreiben eines Analytsensors (4, 5) mit einer elektronischen Schaltung (1) nach einem der Ansprüche 1 bis 8 oder zum Betreiben eines Analytsensorsystems (1, 4, 5) nach Anspruch 9.

## Revendications

1. Circuit électronique (1) configuré pour faire fonctionner un capteur d'analyte (4, 5), tel qu'un capteur de glucose, le circuit ayant au moins une première et une seconde connexion électrique (2, 3) configurées pour être respectivement connectées à une première et une seconde électrode (3, 4) du capteur d'analyte,
dans lequel le circuit électronique (1) a une source de tension (8) et une section conductrice de potentiel commun (6) électriquement fournies sur un potentiel d'un pôle de la source de tension,
dans lequel avec la source de tension (8) un potentiel électrique différent du potentiel de la section conductrice de potentiel commun (6) peut être fourni à la première connexion électrique (2) ; dans lequel la seconde connexion électrique (3) est connectée à la section conductrice de potentiel commun (6) par le biais d'un ou plusieurs trajets de connexion de potentiel commun (10, 11, 20, 21, 22, 23, 30, 31, 32, 33) et dans lequel aucun des trajets de connexion de potentiel commun (10, 11, 20, 21, 22, 23, 30, 31, 32, 33) ne connecte la seconde connexion électrique (3) à la section conductrice de potentiel commun (6) par le biais de moins de trois composants électroniques (12, 13,...) connectés en série ;
**caractérisé en ce qu'**au moins dans un, deux, trois trajets de connexion de potentiel commun (10, 11, 20, 21, 22, 23, 30, 31, 32, 33) ou plus, les composants électroniques (12, 13,...) dans chaque trajet de connexion de potentiel commun (10, 11, 20, 21, 22, 23, 30, 31, 32, 33) sont du même type, le type étant choisi dans un groupe de types, le groupe de types incluant par exemple un composant inductif, un composant capacitif, un composant résistif et/ou un composant de type diode ; et
dans lequel tous les composants électroniques (12, 13,...) du même type dans un trajet de connexion de potentiel commun (10, 11, 20, 21, 22, 23, 30, 31, 32, 33) ont la même spécification nominale concernant leur fonction électrique, telle que la même valeur de résistance nominale, la même valeur de capacité nominale, la même valeur d'inductivité nominale ou la même spécification de diode.

2. Circuit électronique selon la revendication 1, dans lequel dans différents trajets de connexion de potentiel commun (10, 11, 20, 21, 22, 23, 30, 31, 32, 33) des composants électroniques (12, 13) de types différents sont fournis, les types étant choisis parmi un groupe de types, le groupe de types incluant par exemple un composant inductif, un composant capacitif, un composant résistif et/ou un composant de type diode.

3. Circuit électronique selon l'une quelconque des revendications 1 ou 2, dans lequel les composants électroniques (12, 13,...) connectés en série d'un, de deux, de trois trajets de connexion de potentiel commun (10, 11, 20, 21, 22, 23, 30, 31, 32, 33) ou plus, ou tous, sont fournis en tant que composants sur une carte de circuit (36), éventuellement en tant que composants montés en surface (30a,..., 33c).

4. Circuit électronique selon l'une quelconque des revendications 1 à 3, dans lequel tous les composants électroniques (12, 13,...) connectés en série de tous les trajets de connexion de potentiel commun (10, 11, 20, 21, 22, 23, 30, 31, 32, 33) sont fournis sur une carte de circuit (36), éventuellement en tant que composants montés en surface (30a,..., 33c).

5. Circuit électronique selon l'une quelconque des revendications 1 à 4, dans lequel la distance (34, 35) d'un quelconque composant électronique (30a.. 33c) connecté en série dans un, deux, trois trajets de connexion de potentiel commun, ou plus, ou tous, jusqu'à un quelconque autre composant électronique du circuit électronique qui n'est pas un composant électronique connecté en série directement connecté est d'au moins 0,25 mm ou 0,4 mm ou 0,5 mm ou 0,6 mm ou 0,75 mm.

6. Circuit électronique selon l'une quelconque des revendications 1 à 5, dans lequel la distance (34, 35) de tous les composants électroniques (30a.. 33c) connectés en série de tous les trajets de connexion de potentiel commun jusqu'à un quelconque autre composant électronique qui n'est pas un composant électronique connecté en série directement connecté est d'au moins 0,25 mm ou 0,4 mm ou 0,5 mm ou 0,6 mm ou 0,75 mm.

7. Circuit électronique selon l'une quelconque des revendications 1 à 6, dans lequel par un court-circuit de tous les composants électroniques (12, 13,... 33c) connectés en série sauf un dans chaque trajet de connexion de potentiel commun (10, 11, 20, 21, 22, 23, 30, 31, 32, 33) le courant résultant par le biais de la première ou la seconde connexion électrique (2, 3) est limité à moins qu'un seuil prédéfini, le seuil étant inférieur ou égal à 25 µA, 50 µA ou 75 µA.

8. Circuit électronique selon la revendication 7, dans lequel le courant résultant est limité à moins que le seuil prédéfini dans le cas où le potentiel électrique fourni à la première connexion électrique (2) est égal au potentiel maximal disponible dans le circuit électronique tel que par exemple le potentiel fourni par une source de tension ou une batterie (8) qui alimente le circuit électronique (1) et/ou dans le cas où les première et seconde connexions électriques sont court-circuitées p. ex. par un contact électrique des deux électrodes.

9. Système de capteur d'analyte (1, 4, 5) comprenant le circuit électronique (1) selon l'une quelconque des revendications 1 à 8 et un capteur d'analyte (4, 5) tel qu'un capteur de glucose, dans lequel le capteur d'analyte (4, 5) est fourni avec le circuit électronique en tant que kit et le capteur d'analyte (4, 5) et le circuit électronique (1) ne sont pas connectés, ou le capteur d'analyte (4, 5) est connecté au circuit électronique (1).

10. Procédé de fonctionnement d'un capteur d'analyte (4, 5) avec un circuit électronique (1) selon l'une quelconque des revendications 1 à 8 ou de fonctionnement d'un système de capteur d'analyte (1, 4, 5) selon la revendication 9.
